# EUROPÄISCHE PATENTSCHRIFT

(11) **EP 0 812 422 B1**
(45) Veröffentlichungstag und Bekanntmachung des Hinweises auf die Patenterteilung: **08.11.2000**
(21) Anmeldenummer: 96905824.7
(22) Anmeldetag: 29.02.1996
(51) Int. Cl.: G01N 33/58, G01N 33/543, C12Q 1/00, C12Q 1/68, A61K 49/00

(54) **VERFAHREN UND VERBINDUNGEN ZUR DETEKTION VON ANALYTEN MITTELS REMANENZMESSUNG UND DEREN VERWENDUNG**
PROCESS AND COMPOUNDS FOR USE IN DETECTING ANALYTES BY MEASUREMENT OF RESIDUAL MAGNETISM, AND THE USE OF THE SAID COMPOUNDS
PROCEDE ET COMPOSES POUR LA DETECTION D'ANALYTES PAR MESURE DE L'AIMANTATION REMANENTE, ET UTILISATION DESDITS COMPOSES

(30) Priorität: 01.03.1995 DE 19508772
(43) Veröffentlichungstag der Anmeldung: 17.12.1997
(73) Patentinhaber: SCHERING AKTIENGESELLSCHAFT, 13353 Berlin (DE)
(72) Erfinder: WEITSCHIES, Werner, D-10961 Berlin (DE); KÖTITZ, Roman, D-13189 Berlin (DE); TRAHMS, Lutz, D-12103 Berlin (DE); BUNTE, Thomas, D-12307 Berlin (DE)
(86) Internationale Anmeldenummer: EP9600823
(87) Internationale Veröffentlichungsnummer: WO9627133

(56) Entgegenhaltungen:
- EP-A- 0 180 384
- WO-A-91/15243
- DE-A- 3 027 012
- US-A- 4 661 408
- DATABASE WPI Section Ch, Week 9145 Derwent Publications Ltd., London, GB; Class B04, AN 91-329371 [45] XP002008119 & JP,A,03 220 442 (TDK CORP.) , 27.September 1991

## Beschreibung

Die Erfindung betrifft den in den Patentansprüchen gekennzeichneten Gegenstand, daß heißt Verfahren zur qualitativen und/oder quantitativen Detektion von Analyten in Flüssig- und Festphasen mittels Remanenzmessung, die Verwendung dafür geeigneter Verbindungen und deren Verwendung in der Analytik.

Es ist bekannt, daß quantitative Immunoassays sowie andere Bindungsassays (z.B. Rezeptorbindungsassays) es ermöglichen, eine sehr große Anzahl von Substanzen, die auch von biologischer Relevanz sein können, in Proben unterschiedlicher Zusammensetzung zu bestimmen. In der Regel wird hierbei jedoch nur ein Parameter pro Probe in einem Assay bestimmt. Eine aktuelle Übersicht der verschiedenen Verfahren gibt T. Chard [An Introduction to Radioimmunoassay and Related Techniques : Laboratory Techniques in Biochemistry and Molecular Biology, 4. ed., Elsevier Science Publishers, Amsterdam (1990)]. Grundlage aller Bindungsassays ist die hohe Nachweisempfindlichkeit isotopen- oder anders markierter Verbindungen in Kombination mit der großen Spezifität von Ligand-Rezeptorreaktionen.

Die bekannten Assayverfahren haben jedoch folgende Nachteile:
- Die Verfahren für die simultane Bestimmung verschiedener Analyten innerhalb derselben Probe basieren auf der Bindung unterschiedlich radio-, fluoreszenz- oder enzymologisch markierter Sonden an die Analyten. Dabei wird in der Regel nach anschließender Separation und Wäsche die nichtgebundene bzw. gebundene Aktivität der Sonde für die quantitative Bestimmung des Analyten gemessen. Die Menge der einsetzbaren unterschiedlichen Sondenlabel ist dabei stark begrenzt. So treten zum Beispiel im Falle von unterschiedlichen Radioisotopen als Sondenmarkierung sogenannte Überlappungsphänomene auf, die zu einem rapiden Verlust der quantitativen Genauigkeit der individuellen Signale führen. Die Kombination verschiedener Enzyme als Sondenlabel verursacht vergleichbare Probleme, wobei die Durchführbarkeit hier zusätzlich durch die erforderliche Suche nach Reaktionsbedingungen, die die simultane Bestimmung der Enzymreaktionen in einem System erlauben, erschwert wird.
   Beim in der Druckschrift JP3-220442A offenbarten Verfahren wird die Bestimmung eines Antigentiters durch die Messung des Ausmaßes der antikorpervermittelten Aggregation (Agglutination) mit Hilfe eines in der Druckschrift offenbarten Verfahrens zur Messung der Teilchengrößen agglomerierter magnetischer Teilchen durchgeführt. Das Verfahren der Bestimmung der Teilchengröße besteht darin, ein Magnetfeld, welches die ortsfeste sedimentierte Probe durchdringt, zu schalten, und die residuale magnetische Flußdichte der agglomerierten magnetischen Teilchen zu messen.
   Aus der Druckschrift US-4,661,408 sind Ferrofluide bekannt, die superparamagnetische Teilchen aus Chromdioxid mit daran gekoppelten Antikörpern enthalten. Es wird dort beansprucht, daß diese Teilchen sehr kleine intrinsische Relaxationszeiten aufweisen, sie entsprechen deshalb den Anforderungen für die Anwendung in Remanenzmessungen nicht.
- Die Sensitivität der Verfahren ist begrenzt zum Beispiel durch unspezifische Wechselwirkungen zwischen Matrix und Sonde, oder aber durch limitierte Markierungsmöglichkeit der Sonde (geringe spezifische Aktivität).
- Die erfolgreiche Durchführung der Verfahren setzt häufig eine Aufarbeitung des gewonnenen Probenmaterials (z. B. Herstellung von Serum bzw. Plasma aus Vollblut, Extraktion von Proben mit organischen Lösungsmitteln, Anreicherung des Analyten mittels chromatographischer Verfahren u.s.w.) voraus.
- Für die erfolgreiche Durchführung der Verfahren sind Separations- und Waschschritte, die der Trennung von gebundenen und ungebundenen Rezeptoren bzw. Liganden dienen, unerläßlich.
- Für die Durchführung von Radioimmunoassays ist der Einsatz teurer und in der Handhabung aufwendiger strahlender Nuklide erforderlich.
- Die Lagerung der bisher verwendeten Marker bereitet in der Praxis oft Probleme, da sie entweder nicht stabil sind (Radioimmunoassays) und deshalb stets frisch erzeugt werden müssen oder aber empfindlich auf Umgebungseinflüsse reagieren.

Aufgabe der vorliegenden Erfindung war es daher, ein neuartiges Verfahren und Substanzen zu finden, die dem oben genannten Stand der Technik überlegen sind.

Diese Aufgabe wird durch die vorliegende Erfindung gelöst.

Es wurde gefunden, daß die qualitative und/oder quantitative Detektion von Analyten in Flüssig- und/oder Festphasen gelingt, wenn stabile oder quasistabile ferro- oder ferrimagnetische kolloidale heterogenen Substanzen als nachzuweisende magnetische Markierung in Immunoassays oder Bindungsassays eingesetzt werden und die remanente Magnetisierung der Probe als Meßgröße bestimmt wird.

Nachfolgend werden zunächst Verfahren beschrieben, die die Nachteile der bekannten Verfahren überwinden.

Die erfindungsgemäßen Verfahren beruhen auf der Verwendung kolloidaler ferromagnetischer oder ferrimagnetischer Substanzen, im folgenden auch als magnetische Markierung bezeichnet, die mit nachzuweisenden Substanzen - im folgenden auch als Analyte bezeichnet - oder strukturspezifischen Substanzen, die spezifisch an die zu detektierenden Analyte binden, kombiniert werden. Derartige Kombinationen aus magnetischen Markierungen mit Analyten oder strukturspezifischen Substanzen, werden im folgenden auch als magnetische Marker bezeichnet. Durch die Verwendung des Begriffes kolloidale Substanzen wird sowohl der Größenbereich der Teilchen oder Substanzen auf den Größenbereich von Kolloiden, d.h. den Bereich von 1 nm bis ca. 1000 nm, als auch deren Verwendung als dispergierte Phase in einem geeigneten Dispersionsmedium, das zumeist wäßrig ist, beschrieben. Kolloidale Substanzen, die im folgenden auch als Teilchen bezeichnet werden, können zum Zweck der verbesserten Lager- und Transportfähigkeit auch in getrockneter Form oder eingefroren vorliegen, während der Durchführung von Messungen liegen sie jedoch im allgemeinen in flüssiger Phase dispergiertem Zustand vor.

Eine wesentliche Grundlage der Erfindung ist, daß die Zeitabhängigkeit der Magnetisierung ferro- oder ferrimagnetischer kolloidaler Substanzen nach Abschalten eines äußeren magnetisierenden Feldes sehr empfindlich von Material und Form (Anisotropiekonstante des verwendeten Teilchenmaterials), Volumen und der Temperatur der verwendeten Teilchen abhängt. Das ist verursacht durch eine Drehung des internen Magnetisierungsvektors innerhalb der Teilchen. Man bezeichnet diesen Mechanismus als Néelsche Relaxation. Relaxiert die Magnetisierung der Teilchen innerhalb der Meßzeit, so wird das als intrinsischer Superparamagnetismus bezeichnet. Teilchen, deren Néel-Relaxationszeiten wesentlich länger als der Beobachtungszeitraum sind, werden als remanente Teilchen oder auch als stabile Teilchen bezeichnet. Teilchen, deren Néel-Relaxationszeiten in der Größenordnung des Beobachtungszeitraumes sind, werden als quasistabile Teilchen bezeichnet.

Eine weitere wesentliche Grundlage der Erfindung ist es, daß die Magnetisierung einer Gesamtheit frei beweglicher stabiler oder quasistabiler ferro- oder ferrimagnetischer kolloidaler Teilchen nach Abschalten eines äußeren magnetisierenden Feldes innerhalb der Meßzeit durch einen zweiten Mechanismen relaxiert. Dabei kommt es zu einer Drehung des gesamten kolloidalen Teilchens innerhalb der umgebenden Flüssigkeit, wobei die Zeitkonstante vom hydrodynamischen Durchmesser der Teilchen einschließlich der Hülle, der Viskosität der Trägerflüssigkeit und der Temperatur abhängt. Diese Parameter werden hauptsächlich durch die Umgebung der Teilchen bestimmt. Der Mechanismus wird als Brownsche Relaxation oder extrinsischer Superparamagnetismus bezeichnet.

Das erfindungsgemäße Verfahren zur qualitativen und/oder quantitativen Detektion von Analyten in Flüssig- und Festphasen wird durchgeführt, indem
(i) zunächst strukturspezifische Substanzen, welche spezifisch an den zu detektierenden Analyten binden, mit ferrimagnetischen oder ferromagnetischen Substanzen markiert werden und anschließend
(ii) diese magnetisch markierten strukturspezifischen Substanzen zu der zu vermessenden Probe gegeben werden,
(iii) die zu vermessende Probe mittels eines von außen angelegten Magnetfeldes geeigneter Stärke aufmagnetisiert wird und
(iv) nach Abschalten des äußeren Feldes die Remanenz der Magnetisierung der kolloidalen Teilchen (magnetischen Markierung) mittels Magnetfeldsensoren vermessen wird,
wobei die durch spezifische Bindung auftretende Remanenz und deren Ausmaß zur Analyse herangezogen wird.

Die bei den Verfahren des Standes der Technik nur in Ausnahmefällen mögliche Diskriminierung zwischen gebundenen und ungebundenen Markern wird bei dem erfindungsgemäßen Verfahren durch die Ausnutzung der verschwindenden Remanenz (d.h. des extrinsischen Superparamagnetismus) der ungebundenen magnetischen Marker in der flüssigen Phase ermöglicht. Die Gesamtheit der gebundenen magnetischen Marker hingegen zeigt eine - vom Teilchenmaterial abhängige - meßbare Remanenz. Bei der Messung der Remanenz der zu bestimmenden Probe wird also nur der Anteil der gebundenen magnetischen Marker erfaßt. Das Verfahren wird daher nachfolgend auch als Messung der Bindungsremanenz oder Bindungsremanenzmessung bezeichnet und basiert auf der quantitativen Detektion gebundener strukturspezifischer Marker.

Mit anderen Worten, die Bestimmung des Analyten kann auch ohne aufwendige Separations- und Waschschritt erfolgen, da nur die an den Analyten spezifisch gebundenen stabilen oder quasistabilen ferromagnetischen oder ferrimagnetischen Substanzen (die zum Zwecke der Verleihung einer Spezifität mit strukturspezifischen Substanzen kombiniert sind) Remanenz verursachen, wohingegen die Magnetisierung von gleichzeitig in der Probe vorhandenen überschüssigen, ungebundenen stabilen oder quasistabilen ferromagnetischen oder ferrimagnetischen Substanzen (die ebenfalls mit strukturspezifischen Substanzen kombiniert sind) schon vor Beginn der Messung durch extrinsischen Superparamagnetismus abgeklungen ist.

In einer weiteren Ausführungsform der Erfindung ist das Verfahren in der Weise modifiziert, daß anstelle der strukturspezifischen Substanzen die Analyte selbst mit den stabilen oder quasistabilen ferromagnetischen oder ferrimagnetischen Substanzen kombiniert werden, wie dies analog z.B. bei der Durchführung von kompetitiven Radioimmunoassays häufig angewendet wird. Den Proben muß dann noch zusätzlich die strukturspezifische Substanz, welche spezifisch an den zu detektierenden Analyten bindet, zugefügt werden.

Das Verfahren kann aber auch als Multianalytassay, das eine direkte simultane Bestimmung von mehreren verschiedenen Analyten in Flüssigkeiten oder Festsubstanzen ermöglicht, durchgeführt werden. Dazu ist es erforderlich, die Analyten zunächst mit unterschiedlichen ferromagnetischen oder ferrimagnetischen kolloidalen Substanzen mit ausreichend diskreten Koerzitivfeldstärken zu markieren. Während der Bindung der magnetischen Marker wird ein magnetisierendes Feld (primäres Magnetisierungsfeld) angelegt, welches zu einer Ausrichtung aller in der Probe enthaltenen magnetischen Marker entlang ihrer leichten Achse führt. Anschließend wird die Remanenz der Probe bestimmt. In weiteren Schritten wird die Probe mit externen Gegenfeldern (entgegengesetzt zum primären Magnetisierungsfeld), die in ihrer Stärke mit den Koerzitivfeldstärken der magnetischen Markierungen abgestimmt sind, ummagnetisiert. Dadurch wird es möglich, gezielt immer nur diejenigen Markierungen umzuorientieren, deren Koerzitivfeldstärken geringer als das angelegte magnetisierende Feld sind. Zwischen den einzelnen Schritten der Ummagnetisierung wird die Remanenz der Probe jeweils erneut bestimmt.
Aus den gemessenen Remanenzen der Probe bei jedem einzelnen Ummagnetisierungsschritt kann somit der Anteil der verschiedenen gebundenen magnetischen Marker quantifiziert werden.
Durch dieses Verfahren ist es möglich, simultan mehrere Analyten pro Probe zu bestimmen.

Das erfindungsgemäße Verfahren, sowie Varianten des Verfahrens können in der Fertilität, Histokompatibilität, Allergologie, Infektiologie, Hygiene, Genetik, Virologie, Bakteriologie, Toxikologie, Pathologie, Umweltanalytik und medizinischen Diagnostik zum Einsatz kommen.

Der Nachweis der Bindungsremanenz erfolgt mit bekannten, hierfür geeigneten Meßanordnungen, wie zum Beispiel Superconducting Quantum Interference Devices (SQUIDs), wobei zunächst eine Aufmagnetisierung mittels eines geeigneten Magnetfeldes vorgenommen wird und anschließend, nach Abschalten des Feldes, die Messung der Remanenz der magnetisierten strukturspezifischen Substanz oder davon abhängiger Signale durch empfindliche Magnetfeldsensoren, durchgeführt wird.

Während der Messung kann die Probe vorteilhafterweise bewegt werden. Insbesondere vorteilhaft ist eine Modulation des Signals durch Vibration oder Rotation der Probe. Damit wird eine Transformation des dc-Meßsignals in einen höheren Frequenzbereich realisiert.

Zur Messung können - neben den genannten SQUIDs - auch als Gradiometer verschaltete Induktionsspulen, Fluxgate-Magnetometer, Giant-Magnetoresistance-Sensoren oder magnetoresistive Wandler eingesetzt werden.

Bei Verwendung von Sensoren, die dc-Felder messen können (z.B. SQUIDs, Fluxgate-Magnetometer, Giant-Magnetoresistance-Sensoren oder magnetoresistive Wandler) ist eine Messung der Remanenz nach vorheriger Aufmagnetisierung der Probe auch möglich, ohne daß diese bewegt wird.

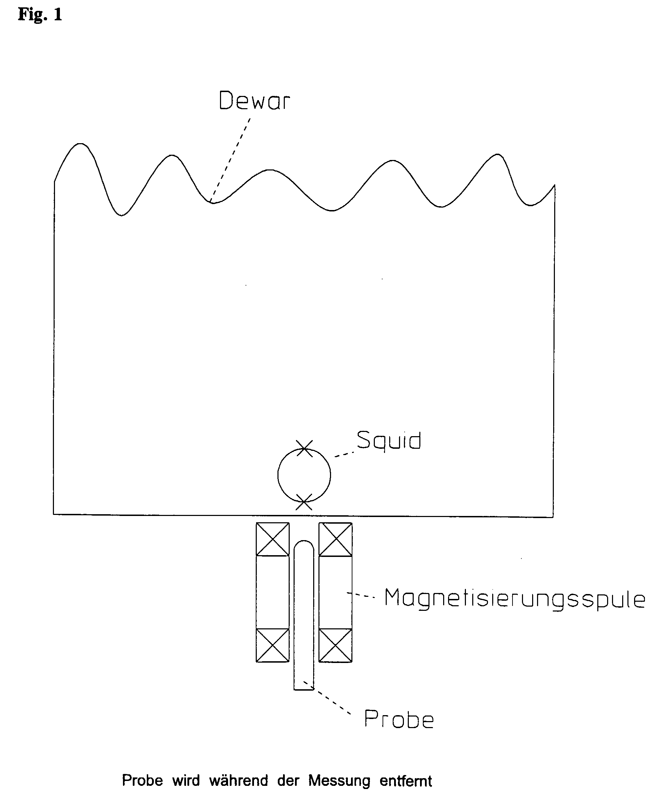

Eine geeignete Meßanordnung ist beispielhaft in Figur 1 abgebildet. Eine derartige Anordnung wurde auch in den nachfolgenden Beispielen verwendet.

Ein weiterer Aspekt der Erfindung betrifft die Verwendung von Verbindungen zur Messung der Bindungsremanenz. Geeignete Verbindungen bestehen aus kolloidalen Suspensionen ferrimagnetischer oder ferromagnetischer Substanzen und strukturspezifischen Substanzen oder Analyten.

Unter strukturspezifischen Substanzen sind im Rahmen der vorliegenden Erfindung alle Substanzen zu verstehen, die spezifisch an die gewünschte Struktur binden, wie z.B. Antikörper, Antikörperfragmente, Biotin oder Biotin spezifisch bindende Substanzen wie Avidin oder Streptavidin, spezifisch an Rezeptoren bindende Agonisten wie Zytokine, Lymphokine, Endotheline oder deren Antagonisten, spezifische Peptide und Proteine, Rezeptoren, Enzyme, Enzymsubstrate, Nukleotide, Ribonukleinsäuren, Desoxyribonukleinsäuren, Kohlenhydrate, Lipoproteine u.s.w..

Darunter sind Substanzen bevorzugt, deren Bindungskonstante im Bereich von 10⁵ - 10¹⁵ (mol/l)⁻¹ liegt, insbesondere Substanzen deren Bindungskonstante im Bereich von 10⁷ - 10¹⁵ (mol/l)⁻¹ liegt.

Als ferro- oder ferrimagnetische kolloidale Substanzen können alle ferromagnetischen und ferrimagnetischen Substanzen verwendet werden, deren intrinsische Néel-Relaxationszeit größer oder gleich der Meßzeit ist und die somit stabil bzw. quasistabil sind.

Besonders geeignet sind alle ferromagnetischen und ferrimagnetischen Substanzen, deren Relaxationszeit bei 20°C länger als 10⁻⁴ Sekunden ist. Insbesondere geeignet sind alle ferromagnetischen und ferrimagnetischen Substanzen, deren Relaxationszeit länger als 1 Sekunde ist.

Die ferromagnetischen und ferrimagnetischen Substanzen können vorteilhafterweise mit einer Hülle aus oligomeren oder polymeren Kohlenhydraten, Proteinen, Peptiden, Nukleotiden, Tensiden, synthetischen Polymeren und/oder Lipiden stabilisiert sein.

Die Partikelgrößen der ferromagnetischen und ferrimagnetischen Substanzen liegen vorteilhafterweise zwischen 1 nm und 1000 nm. Insbesonders bevorzugt sind Partikelgrößen zwischen 2 nm und 500 nm.(Die Angaben hinsichtlich der Partikelgröße beziehen sich auf den hydrodynamischer Durchmesser.)

Als ferro- oder ferrimagnetische Substanzen sind insbesondere stabile oder quasistabile kolloidale Teilchen aus Eisenoxiden (Fe₃O₄, γ-Fe₂O₃), Bariumferrit, Strontiumferrit, Reineisen, Chromdioxid, Nickel, Kobalt sowie Eisenoxiden mit Mangan-, Kupfer-, Nickel- oder Kobaltzusätzen (wie z.B. in DE 30 27 012 und DE 41 16 093 beschrieben) geeignet.

Die Herstellung der erfindungsgemäß einsetzbaren Verbindungen, bestehend aus stabilen oder quasistabilen ferromagnetischen oder ferrimagnetischen Substanzen, die mit strukturspezifischen Substanzen oder Analyten verbunden sind, erfolgt mit Hilfe der im Bereich der Immuno-, Peptid- und Proteinchemie geläufigen Verfahren. Dabei wird die strukturspezifische Substanz oder der Analyt über kovalente Bindungen mit den die stabilisierende Hülle der ferromagnetischen oder ferrimagnetischen Substanzen bildenden Substanzen verknüpft. Als stabilisierende Hülle kommen z.B. Kohlenhydrate, Peptide, Nukleotide, Proteine, Lipide, Tenside oder Polymere infrage. Besonders geeignete Verknüpfungsmethoden sind z.B. die Aktivierung und Kopplung mittels Carbodiimiden [Jakoby and Wilchek, eds., (1974) Methods Enzymol 34], die Bildung von Schiff'schen Basen nach Einwirkung von Periodaten auf Kohlenhydrate enthaltende Verbindungen (Wichek and Bayer, eds., Methods Enzym 184:177), die gegebenenfalls zur weiteren Stabilisierung anschließend noch reduziert werden, die Kopplung mittels Glutardialdehyd [Heitzmann and Richards, Proc. Natl. Acad. Sci. USA, 71 (1974) 3537], die Quervernetzung bromoacetylierter Teilchen mit thiolylierten Substanzen [Angerer et al., Cell 9 (1976) 81], sowie die reduktive Alkylierung [Bayer et al., J. Histochem. Cytochem. 24 (1976) 933].

Es können auch ferromagnetische oder ferrimagnetische kolloidale Teilchen, mit einer stabilisierenden Hülle aus der strukturspezifischen Substanz oder dem Analyten selbst hergestellt werden, indem die Teilchen nach Herstellung entweder direkt in eine Lösung der strukturspezifischen Substanz oder des Analyten, gegebenenfalls in Gegenwart weiterer Hilfsstoffe wie z.B. Proteine, Kohlenhydrate sowie natürliche, synthetische oder partialsynthetische oberflächenaktive Substanzen usw. gebracht werden, bzw. direkt in Gegenwart der strukturspezifischen Substanzen oder Analyten hergestellt werden.

Zum Zweck der Durchführung von Multianalytassays können auch Mischungen von Verbindungen, die aus mehreren unterschiedlichen magnetischen Markern bestehen, wobei die unterschiedlichen magnetischen Marker aus Kombinationen unterschiedlicher strukturspezifischer Substanzen oder nachzuweisender Analyte mit unterschiedlichen ferromagnetischen oder ferrimagnetischen Substanzen bestehen, zur Anwendung kommen. Grundlage dieser erfindungsgemäßen Verwendung von Kombinationen unterschiedlicher magnetischer Marker ist es, daß als magnetische Markierung für die jeweiligen strukturspezifischen Substanzen oder nachzuweisenden Analyte ferromagnetische oder ferrimagnetische Substanzen mit unterscheidbaren Koerzitivfeldstärken (H_{c}) verwendet werden, wobei die Parameter Koerzitivfeldstärke (H_{c}) und remanente Magnetisierung (M_{R}) für die unterschiedlichen magnetischen Markierungen in bekannter Weise vorab separat bestimmbar und somit bekannt sind.

Die mit den ferromagnetischen oder ferrimagnetischen kolloidalen Substanzen markierten strukturspezifischen Substanzen können auch in getrockneter Form (z.B. als Lyophilisate), gegebenenfalls in Kombination mit weiteren Hilfsstoffen, die die Trocknung erleichtern oder die Stabilität des getrockneten Produkts erhöhen, vorliegen. Die Herstellung der gebrauchsfertigen Mittel aus derartigen Lyophilisaten erfolgt dann durch Resuspendieren in einem geeigneten Suspensionsmedium unmittelbar vor der Anwendung.

Ein weiterer Aspekt der Erfindung betrifft somit Mittel zur Bindungsremanenzmessung enthaltend ferro- oder ferrimagnetische kolloidale Substanzen in einem geeigneten Suspensionsmedium.

Als Suspensionsmedien kommen alle Flüssigkeiten in Betracht in denen die kolloidalen Teilchen frei beweglich sind. Soll die Messungen ohne Separations- oder Waschschritte durchgeführt werden, muß die Viskosität des verwendeten Suspensionsmediums mit der Néel-Relaxationszeit der ferromagnetischen und ferrimagnetischen Substanzen und der Meßzeit abgestimmt werden, da das Suspensionsmedium wesentlich die Zeitkonstante der Brownschen Relaxation bestimmt. Andernfalls kann z.B. bei Verwendung von Teilchen mit kurzer Relaxationszeit (z.B. 0,01 Sekunden) in hochviskosen Suspensionsmedien (z.B. 80% Glycerol) und kurzer Meßzeit (z.B. 0,0001 Sekunden nach Abschalten des äußeren Feldes) die Brownsche Relaxation (extrinsischer Superparamagnetismus) derart verlangsamt sein, daß nicht mehr zwischen gebundenen und ungebundenen strukturspezifischen Markern unterschieden werden kann. Es ist im allgemeinen vorteilhaft, Suspensionsmedien einer Viskosität, die kleiner als 100 mPas ist, zu verwenden.

Als Suspensionsmedium geeignet sind Wasser, wäßrige Lösungen oberflächenaktiver Hilfsstoffe, wie z.B. Tenside oder oligomere oder polymere Kohlenhydrate und Proteine, sowie Mischungen aus Wasser mit Alkoholen wie z.B. Glycerol und Polyethylenglykol, wobei für Injektionszwecke geeignetes Wasser bevorzugt ist. Die Suspensionsmedien können zusätzlich den osmotischen Druck verändernde Hilfsstoffe, wie z.B. Kochsalz, enthalten. Des weiteren können den pH-Wert bestimmende Puffersubstanzen, wie z.B. Phosphate, enthalten sein.

Ein weiterer Gegenstand der Erfindung ist die Verwendung der Verbindungen in den erfindungsgemäßen Verfahren zur Messung der Bindungsremanenz.

Aufgrund des auf physikalischen Mechanismen beruhenden Bindungsnachweises können unspezifische Meßsignale (Matrixphänomene) weitgehend ausgeschlossen werden. Die Spezifität des Verfahrens hängt somit nur noch von der "wahren" Spezifität der strukturspezifischen Substanz (Kreuzreaktivität von Antikörpern, unspezifische Bindung von Liganden) ab.

Aufgrund der hohen Sensitivität des erfindungsgemäßen Verfahrens werden die sonst üblichen Detektionsgrenzen von Bindungsassays mühelos unterschritten.

Im Gegensatz zu bekannten Methoden (JP-235774 und WO 91/15243) wird bei dem erfindungsgemäßen Verfahren nicht die statische Magnetisierung in Anwesenheit eines äußeren magnetisierenden Feldes, sondern in Abwesenheit eines Magnetfeldes die Bindungsremanenz oder davon abhängige Signale gemessen. Erst dadurch werden Informationen über den Bindungszustand der Marker zugänglich.

Desweiteren wird dadurch auch eine Beeinflußung des Meßsignals durch dia- oder paramagnetische Komponenten beziehungsweise Verunreinigungen vermieden, was zu einer weiteren Steigerung der Meßempfindlichkeit beiträgt.

Die erfindungsgemäßen Verfahren zur Bindungsremanenzmessung können des weiteren auch dazu verwendet werden, den Aufenthaltsort ferro- oder ferrimagnetischer Substanzen in vivo nachzuweisen. Hierbei ist von besonderem Vorteil, daß zur Bestimmung der Bindungsremanenz ferro- oder ferrimagnetische Substanzen verwendet werden können und damit die am Menschen besonders kritische Anwendung radioaktiver Isotope vermieden wird, wobei die Empfindlichkeit des erfindungsgemäßen Verfahrens die von gebräuchlichen nuklearmedizinischen Verfahren der Gammaszintigraphie oder Positronen-Emissions-Tomographie erreicht. Darüber hinaus ist eine vorherige Abtrennung von im Blut zirkulierenden ungebundenen Marker überflüssig, da dieser (im Gegensatz zu radiodiagnostischen Methoden) kein "Störsignal" verursacht und somit die Detektion des spezifisch gebundenen Markers nicht beeinflußt.

Erfindungsgemäß wird hierfür dem Patienten eine Suspension stabiler oder quasistabiler ferro- oder ferrimagnetischer Substanzen verabreicht. Als Applikationswege sind die lokale Auftragung, die perorale Applikation und alle Formen der parenteralen Applikation geeignet. Besonders geeignet sind intravasale Applikationsformen.

Kolloidale Substanzen werden im Organismus verteilt, wobei das Verteilungsmuster im Körper vom Applikationsweg sowie von verschiedenen anderen pharmakokinetischen Parametern beeinflußt wird. Die ortsaufgelöste Bestimmung der Bindungsremanenz zu unterschiedlichen Zeiten nach Applikation stabiler oder quasistabiler ferro- oder ferrimagnetischer Substanzen kann dazu dienen, pathologische Zustände im Körper, die sowohl durch ungewöhnliche Anreicherungen oder fehlendes Auftreten von Anreicherungen gekennzeichnet sind, festzustellen.

Besonders vorteilhaft kann zur Darstellung pathologischer Strukturen im menschlichen Körper die erfindungsgemäße Verwendung von mit strukturspezifischen Substanzen kombinierten stabilen oder quasistabilen ferro- oder ferrimagnetischen Substanzen (magnetische Marker) sein. Hier führt die spezifische Bindung der magnetischen Marker an die pathologische Struktur zu einem spezifischen Signal, das erfindungsgemäß mit den für die Durchführung von Bindungsassays beschriebenen Verfahren der Bindungsremanenz *in vivo* bestimmt werden kann.

Als Beispiel für eine erfindungsgemäße Anwendung von stabilen oder quasistabilen ferro- oder ferrimagnetischen Substanzen in Kombination mit den Verfahren der Bindungsremanenzmessung, sei die Detektion von Tumoren im Körper durch Verwendung von tumorspezifischen magnetischen Markern genannt. Tumorspezifische Marker können z.B. Kombinationen von stabilen oder quasistabilen ferro- oder ferrimagnetischen Substanzen mit tumorspezifischen Substanzen, wie z.B. Antikörpern, Antikörperfragmenten, Peptiden, Rezeptoren, Proteinen, Nukleinsäuren, Oligonukleotiden, monomeren, oligomeren oder polymeren Kohlenhydraten, sein.

Weitere Beispiele für mögliche Anwendungen sind die Darstellung von Thromben, atherosklerotischen Plaques, entzündlichen Reaktionen, rheumatischen oder rheumatoiden Veränderungen, wobei jeweils vorteilhafterweise erfindungsgemäße Kombinationen aus stabilen oder quasistabilen ferro- oder ferrimagnetischen Substanzen mit für die jeweilige pathologische Struktur spezifischen Substanzen als magnetische Marker eingesetzt werden.

Die *In vivo*-Messung der räumlichen Verteilung einem Menschen applizierter stabiler oder quasistabiler magnetischer Marker, erfolgt erfindungsgemäß nach zwei Varianten:
1. Erzeugung eines möglichst homogen Magnetfeldes in der interessierenden Körperregion, Abschalten des Feldes und Messung der räumlichen Verteilung der Bindungsremanenz mittels eines SQUID-Multikanalsensors, wie er z.B. für biomagnetische Untersuchungen eingesetzt wird [vgl. D. Drung, IEEE Trans. Appl. Supercond., 5 (1995) 2112-2117]. Dieser sollte das Meßobjekt möglichst vollständig umschließen. Zur Erzeugung ausreichender Meßinformation ist eine mehrfache Meßdurchführung unter sequentieller Abrasterung des Meßobjektes vorteilhaft.
2. Sequentielle Erzeugung eines räumlich begrenzten lokalen Feldes, Abschalten des Feldes und Messung der räumlichen der Bindungsremanenz mittels eines Einkanalsensors. Die Verwendung eines Multikanalsensors ist ebenfalls möglich.

Bei beiden Methoden ist zur Gewinnung maximaler Information sowohl die Magnetisierung des Meßobjektes als auch die Messung des resultierenden Magnetfeldes in allen drei Raumrichtungen zu bevorzugen.

Die Auswertung der Messdaten kann durch ein geeignetes Approximationsverfahren erfolgen. So kann man z.B. das Modell des magnetischen Dipols, Multipols oder Multi-Dipols zugrunde legen. Die speziellen Parameter des Modells, insbesondere die Orte der Dipole oder Multipole, werden dann mittels des Approximationsverfahren gefunden, das die Abweichungen zwischen Meßdaten und Modellparametern minimiert. Aus diesen Parametern läßt sich die räumliche Verteilung der magnetisierten Teilchen in an sich bekannter Weise ermitteln.

Eine analoge Vorgehensweise ist bekannt und bewährt bei der Analyse der Magnetfelder von bioelektrischen Strömen.

Für die Messung der Bindungsremanenz *in vivo* eignen sich grundsätzlich dieselben Substanzen - bzw. daraus bereitete Mittel - wie sie auch bei *In vitro*-Untersuchungen zum Einsatz kommen.

Besonders geeignet für eine *In vivo*-Anwendung sind magnetische Markierungen, die biologisch abbaubar und verträglich sind. Dies trifft insbesondere für magnetische Markierungen zu, die aus Eisenoxiden oder aus Kombinationen von Eisenoxiden mit Mangan oder Kobalt bestehen. Magnetische Partikel, an die nach den genannten Verfahren strukturspezifische Substanzen geknüpft werden können, finden z.B. in der Kernspintomographie Anwendung und werden u.a. in der WO 92/12735 der WO 92/22586 sowie der EP 0 186 616 beschrieben. Ein weitere Aspekt der Erfindung betrifft somit die Verwendung von magnetischen Markierungen in einem *In vivo*-Verfahren zur Bindungsremanezmessung.

Unter strukturspezifischen Substanzen sind im Zusammenhang mit der *In vivo*-Anwendung der Bindungsremanenzmessung insbesondere alle Substanzen zu verstehen, die spezifisch an nachzuweisende Strukturen des menschlichen Körpers binden. Besonders geeignet sind Antikörper, Antikörperfragmente, spezifisch an Rezeptoren bindende Agonisten oder deren Antagonisten, spezifische Peptide und Proteine, Rezeptoren, Enzyme, Enzymsubstrate, Nukleotide, Ribonukleinsäuren, Desoxyribonukleinsäuren, Kohlenhydrate oder Lipoproteine. Von den an Rezeptoren bindenden Agonisten sind insbesondere Zytokine, Lymphokine oder Endotheline geeignet.

Gut geeignet sind alle strukturspezifischen Substanzen, die eine Bindungskonstante im Bereich von 10⁵ - 10¹⁵ (mol/l)⁻¹ aufweisen. Insbesonders geeignet sind alle strukturspezifischen Substanzen, die eine Bindungskonstante im Bereich von 10⁷ - 10¹⁵ (mol/l)⁻¹ aufweisen.

Die nachfolgenden Beispiele dienen der näheren Erläuterung des Erfindungsgegenstandes, ohne ihn auf diese beschränken zu wollen.

### Beispiel 1

100 µg eines monoklonalen Antikörpers gegen Collagen III, im folgenden Anti-Collagen III genannt, werden in 500 µl 0,1 M Natriumhydrogencarbonat-Lösung gelöst. 1 ml dextrangecoatete Magnetitsuspension (Meito Sangyo) mit 1 Mol Fe/l und einer Teilchengröße von ca. 40 nm (hydrodynamischer Durchmesser) wird über eine Sephadex Säule (Pharmacia PD 10) mit 0,1 M Natriumhydrogencarbonat umgepuffert. Zu der Suspension werden 0,5 ml 10 mmol Natriumperiodat-Lösung gegeben. Die Lösung wird 2 Stunden im Dunkeln stehengelassen. Anschließend wird über eine PD 10 mit 0,1 M Natriumhydrogencarbonat-Lösung eluiert. Zu der Suspension wird die Anti-Collagen III-Lösung gegeben. Die Mischung wird 3 Stunden bei 4°C im Dunkeln stehengelassen. Anschließend werden 5 mg NaBH₄ als Festsubstanz zugegeben und kurz geschwenkt. Die Mischung wird 8 Stunden bei 4 °C im Dunkeln stehengelassen. Anschließend werden die magnetitmarkierten Anti-Collagen III (im folgenden Mag-Anti-Collagen III genannt) über eine PD 10 Säule mit phosphatgepufferter Kochsalzlösung (PBS, pH 7,4) eluiert.

Eine Lösung von 5 µg Collagen III in 200 µl Puffer [phosphatgepufferte Kochsalzlösung (PBS)] wird in einem Probengefäß aus Polystyrol inkubiert. (Das Probengefäß dient dabei als feste Phase, an der ein Teil des Collagens fixiert wird.) Anschließend wird die Flüssigphase verworfen. Das Probengefäß wird dreimal mit phosphatgepufferte Kochsalzlösung, enthaltend 0,1 % Tween® 20 (nachfolgend als PBST bezeichnet), gespült, um nicht fixiertes Collagen auszuwaschen. In das Probengefäß werden dann 5 µl Mag-Anti-Collagen III gelöst in 200 µl PBST gegeben. Es wird 1 Stunde bei Raumtemperatur inkubiert. Anschließend wird die Probe in einer magnetisch abgeschirmten Kammer in einem Feld der Stärke 2 mT 4 cm unterhalb des Squid-Detektors magnetisiert (siehe Figur 1). Nach Abschalten des Magnetfeldes wird die Probe vermessen. Dazu wird die Probe während der Messung aus der Magnetisierungsspule entfernt. Aus der Differenz der - nach Abschalten des Magnetfeldes - ermittelten magnetischen Flußdichten vor und nach Entfernung der Probe aus dem Messfeld ergibt sich die Remanenz. Im Falle des vorliegenden Beispiels konnte eine Remanenz festgestellt werden.

### Beispiel 2

Eine Lösung von 5 µg Collagen V in 200 µl PBS Puffer pH 7,4 wird in einem Probengefäß aus Polystyrol inkubiert. Anschließend wird die Flüssigphase verworfen. Das Probengefäß wird dreimal mit PBST Waschpuffer pH 7,4 gespült. Zu der Probe werden 5 µl Mag-Anti-Collagen III, hergestellt nach Beispiel 1, in 200 µl PBST gegeben. Es wird 1 Stunde bei Raumtemperatur inkubiert. Anschließend wird die Probe in einer magnetisch abgeschirmten Kammer in einem Feld der Stärke 2 mT 4 cm unterhalb des SQUID-Detektors magnetisiert (siehe Figur 1). Nach Abschalten des Magnetisierungsfeldes wird die Probe vermessen. Dazu wird die Probe während der Messung aus der Magnetisierungsspule entfernt. Bei der Collagen V enthaltenden Probe ist keine Remanenz feststellbar.

### Beispiel 3

Aus 10 ml einer 1,9 mg/ml Collagen III-Lösung in PBS (pH 7,4) werden jeweils 5 ml der folgenden Verdünnungen hergestellt:
1000 ng/ml, 100 ng/ml, 10 ng/ml

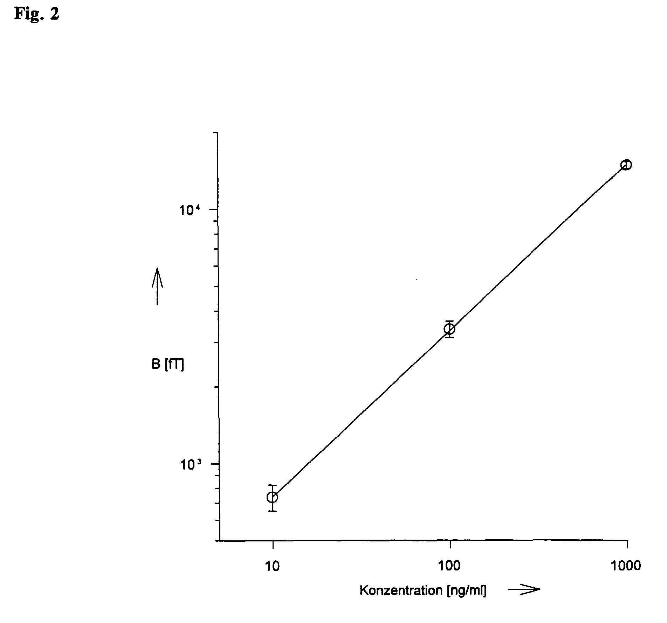

Von jeder Verdünnung werden jeweils 1 ml in jeweils einPolystyrolröhrchen (Fassungsvolumen 2,5 ml) pipettiert. Die drei Probenröhrchen werden 1 Stunde bei 37°C inhibiert. Danach wird der Inhalt der Röhrchen verworfen. Die Röhrchen werden 3mal mit je 1 ml PBST gewaschen.
In jedes Röhrchen werden 1 ml einer 1 : 100-Verdünnung des Magnetit markierten Antikörpers, hergestellt gemäß Beispiel 1, gegeben. Die Röhrchen werden 1 Stunde bei Raumtemperatur stehengelassen. Danach werden die Proben mit der in Figur 1 skizzierten Meßanordnung magnetisiert (2 mT) und nach Abschalten des magnetisierenden Feldes werden die Proben vermessen. Dazu werden die Proben während der Messung aus der Magnetisierungsspule entfernt. Die Auswertung der gemessenen Signale ist ein Maß für die Bindungsremanenz und ergibt den in Figur 2 wiedergegebenen Zusammenhang.

## Patentansprüche

1. Verfahren zur qualitativen und/oder quantitativen Detektion von Analyten, dadurch gekennzeichnet, daß stabile oder quasistabile ferro- oder ferrimagnetische kolloidale Substanzen als nachzuweisende magnetische Markierung in heterogenen Immunoassays eingesetzt werden und die remanente Magnetisierung der Probe als Meßgröße bestimmt wird.

2. Verfahren zur qualitativen und/oder quantitativen Detektion von Analyten in heterogenen Immunoassays, dadurch gekennzeichnet, daß gebundene magnetische Marker zum Zeitpunkt der Messung in ihrer Gesamtheit eine remante Magnetisierung der Probe bewirken, während die Magnetisierung ebenfalls in der Probe vorhandener ungebundener magnetischer Marker zum Zeitpunkt der Messung in ihrer Gesamtheit durch extrinsischen Superparamagnetismus abgeklungen ist.

3. Verfahren zur qualitativen und/oder quantitativen Detektion von Analyten in heterogenen Immunoassays, dadurch gekennzeichnet, daß
(i) zunächst strukturspezifische Substanzen, welche spezifisch an den zu detektierenden Analyten binden, mit kolloidalen ferrimagnetischen oder ferromagnetischen Substanzen markiert werden und anschließend
(ii) diese magnetisch markierten strukturspezifischen Substanzen zu der zu vermessenden Probe gegeben werden,
(iii) die zu vermessende Probe mittels eines von außen angelegten Magnetfeldes geeigneter Stärke aufmagnetisiert wird und
(iv) nach Abschalten des äußeren Feldes die Remanenz der Magnetisierung der kolloidalen Teilchen mittels Magnetfeldsensoren vermessen wird, wobei die durch spezifische Bindung auftretende Remanenz und deren Ausmaß zur Analyse herangezogen wird.

4. Verfahren nach Anspruch 3, dadurch gekennzeichnet, daß anstelle der strukturspezifischen Substanzen nachzuweisende Analyte mit ferrimagnetischen oder ferromagnetischen Substanzen markiert werden und den zu vermessenden Proben die strukturspezifischen Substanzen, welche spezifisch an den zu detektierenden Analyten binden, zugesetzt werden.

5. Verfahren gemäß den Ansprüchen 3 oder 4, dadurch gekennzeichnet, daß die strukturspezifischen Substanzen Antikörper, Antikörperfragmente, Biotin oder Biotin spezifisch bindende Substanzen, Avidin oder Streptavidin, spezifisch an Rezeptoren bindende Agonisten oder deren Antagonisten, spezifische Peptide und Proteine, Rezeptoren, Enzyme, Enzymsubstrate, Nukleotide, Ribonukleinsäuren, Desoxyribonukleinsäuren, Kohlenhydrate oder Lipoproteine sind.

6. Verfahren gemäß Anspruch 5, dadurch gekennzeichnet, daß die spezifisch an Rezeptoren bindenden Agonisten oder Antagonisten Zytokine, Lymphokine, Endotheline oder deren Antagonisten sind.

7. Verfahren gemäß den Ansprüchen 3, 4, 5 oder 6, dadurch gekennzeichnet, daß die strukturspezifischen Substanzen eine Bindungskonstante im Bereich von 10⁵ - 10¹⁵ (mol/l)⁻¹ haben.

8. Verfahren nach einem der Ansprüche 1 bis 7, dadurch gekennzeichnet, daß die Probe während der Messung bewegt und damit das Probensignal moduliert wird.

9. Verfahren nach einem der Ansprüche 1 bis 8, dadurch gekennzeichnet, daß als Magnetfeldsensoren als Gradiometer geschaltete Induktionsspulen, Fluxgate-Magnetometer, Giant-Magnetoresistance-Sensoren oder magnetoresistive Wandler eingesetzt werden.

10. Verfahren nach einem der Ansprüche 1 bis 8, dadurch gekennzeichnet, daß als Magnetfeldsensoren SQUIDs eingesetzt werden.

11. Verfahren gemäß den Ansprüchen 1 bis 10, dadurch gekennzeichnet, daß durch stufenweise Magnetisierung der zu vermessenden Probe eine simultane Bestimmung von mehreren verschiedenen Analyten durchgeführt wird.

12. Verfahren gemäß Anspruch 11, dadurch gekennzeichnet, daß für eine simultane quantitative Bestimmung von Analyten unterschiedliche ferromagnetische oder ferrimagnetische Substanzen mit diskreten Koerzitivfeldstärken verwendet werden.

13. Verfahren gemäß den Ansprüchen 1 bis 12, dadurch gekennzeichnet, daß die intrinsische Néel-Relaxationszeit der verwendeten ferromagnetischen und ferrimagnetischen Substanzen größer als die Messzeit ist.

14. Verfahren gemäß Anspruch 13, dadurch gekennzeichnet, daß die Néelsche Relaxationszeit der verwendeten ferromagnetischen und ferrimagnetischen Substanzen bei 20°C länger als 10⁻⁴ Sekunden ist.

15. Verfahren gemäß den Ansprüchen 1 bis 14, dadurch gekennzeichnet, daß die ferromagnetischen und ferrimagnetischen Substanzen eine Partikelgröße im Bereich von 1 bis 1000 nm aufweisen.

16. Verfahren gemäß den Ansprüchen 1 bis 15, dadurch gekennzeichnet, daß die ferromagnetischen und ferrimagnetischen Substanzen mit einer Hülle aus oligomeren oder polymeren Kohlenhydraten, Proteinen, Peptiden, Nukleotiden, Tensiden, synthetischen Polymeren und/ oder Lipiden stabilisiert sind.

17. Verwendung von Verbindungen in Verfahren nach den Ansprüchen 1-16, dadurch gekennzeichnet, daß die Verbindungen aus Kombinationen von stabilen oder quasistabilen ferrimagnetischen oder ferromagnetischen Substanzen mit strukturspezifischen Substanzen bestehen.

18. Verwendung von Verbindungen gemäß Anspruch 17, dadurch gekennzeichnet, daß die ferrimagnetischen oder ferromagnetischen Teilchen eine Néel-Relaxationszeit aufweisen, die länger als 10⁻⁴ Sekunden ist.

19. Verwendung von Verbindungen gemäß Anspruch 17, dadurch gekennzeichnet, daß die strukturspezifischen Substanzen Antikörper, Antikörperfragmente, spezifisch an Rezeptoren bindende Agonisten, Zytokine, Lymphokine, Endotheline oder deren Antagonisten, sonstige spezifische Peptide und Proteine, Rezeptoren, Enzyme, Enzymsubstrate, Nukleotide, Ribonukleinsäuren, Desoxyribonukleinsäuren, Kohlenhydrate oder Lipoproteine sind.

20. Verwendung von Verbindungen gemäß Anspruch 17, dadurch gekennzeichnet, daß die ferromagnetischen oder ferrimagnetischen Substanzen stabile oder quasistabile kolloidale Teilchen aus Eisenoxiden, Bariumferrit, Strontiumferrit, Reineisen, Chromdioxid, Nickel, Kobalt sowie Eisenoxiden mit Mangan-, Kupfer-, Nickel- oder Kobaltzusätzen sind.

21. Verwendung von Mitteln in Verfahren nach den Ansprüchen 11 oder 12, dadurch gekennzeichnet, daß sie mehrere ferromagnetische oder ferrimagnetische Substanzen mit unterschiedlichen Koerzitivfeldstärken enthalten.

22. Verwendung der Verfahren gemäß den Ansprüchen 1 - 16 in der Fertilität, Histokompatibilität, Allergologie, Infektiologie, Hygiene, Genetik, Virologie, Bakteriologie, Toxikologie, Pathologie, Umweltanalytik oder medizinischen Diagnostik.

## Claims

1. Method for the qualitative and/or quantitative detection of analytes, characterized in that stable or quasi-stable ferro- or ferrimagnetic colloidal substances are used as magnetic marking to be picked up in heterogeneous immunoassays, and the remanent magnetization of the sample is determined as a measured variable.

2. Method for the qualitative and/or quantitative detection of analytes in heterogeneous immunoassays, characterized in that bound magnetic markers cause remanent magnetization of the sample in their entirety at the time of the measurement, while the magnetization of unbound magnetic markers also present in the sample is attenuated in its entirety by extrinsic superparamagnetism at the time of the measurement.

3. Method for the qualitative and/or quantitative detection of analytes in heterogeneous immunoassays, characterized in that
(i) structure-specific substances, which bind specifically to the analytes to be detected, are firstly marked with colloidal ferrimagnetic or ferromagnetic substances, and subsequently
(ii) these magnetically marked structure-specific substances are added to the sample to be measured,
(iii) the sample to be measured is magnetized using an externally applied magnetic field of suitable strength, and
(iv) after the external field is turned off, the remanence of the magnetization of the colloidal particles is measured using magnetic field sensors,
the remanence which occurs through specific binding, and its extent, being employed for analysis.

4. Method according to Claim 3, characterized in that, instead of the structure-specific substances, analytes to be picked up are marked with ferrimagnetic or ferromagnetic substances and the structure-specific substances, which bind specifically to the analytes to be detected, are added to the samples to be measured.

5. Method according to one of Claims 3 and 4, characterized in that the structure-specific substances are antibodies, antibody fragments, biotin or substances which specifically bind biotin, avidin or streptavidin, agonists which bind specifically to receptors, or antagonists thereof, specific peptides and proteins, receptors, enzymes, enzyme substrates, nucleotides, ribonucleic acids, deoxyribonucleic acids, carbohydrates or lipoproteins.

6. Method according to Claim 5, characterized in that the agonists which bind specifically to receptors, or antagonists, are cytokines, lymphokines, endothelins or antagonists thereof.

7. Method according to Claims 3, 4, 5 or 6, characterized in that the structure-specific substances have a binding constant in the 10⁵-10¹⁵ (mol/l)⁻¹ range.

8. Method according to one of Claims 1 to 7, characterized in that the sample is moved during the measurement and the sample signal is thereby modulated.

9. Method according to one of Claims 1 to 8, characterized in that induction coils connected as a gradiometer, flux gate magnetometers, giant magnetoresistance sensors or magnetoresistive transducers are used as the magnetic field sensors.

10. Method according to one of Claims 1 to 8, characterized in that SQUIDs are used as magnetic field sensors.

11. Method according to one of Claims 1 to 10, characterized in that simultaneous determination of a plurality of different analytes is carried out by stepwise magnetization of the sample to be measured.

12. Method according to Claim 11, characterized in that different ferromagnetic or ferrimagnetic substances with discrete coercive field strengths are used for simultaneous quantitative determination of analytes.

13. Method according to Claims 1 to 12, characterized in that the Néel relaxation time of the ferromagnetic and ferrimagnetic substances used is greater than the measurement time.

14. Method according to Claim 13, characterized in that the intrinsic Néel relaxation time of the ferromagnetic and ferrimagnetic substances used at 20°C is longer than 10⁻⁴ seconds.

15. Method according to Claims 1 to 14, characterized in that the ferromagnetic and ferrimagnetic substances have a particle size in the 1 to 1000 nm range.

16. Method according to Claims 1 to 15, characterized in that the ferromagnetic and ferrimagnetic substances are stabilized with an envelope of oligomeric or polymeric carbohydrates, proteins, peptides, nucleotides, surfactants, synthetic polymers and/or lipids.

17. Use of compounds in methods according to one of Claims 1-16, characterized in that the compounds consist of combinations of stable or quasi-stable ferrimagnetic or ferromagnetic substances with structure-specific substances.

18. Use of compounds according to Claim 17, characterized in that the ferrimagnetic or ferromagnetic particles have a Néel relaxation time which is longer than 10⁻⁴ seconds.

19. Use of compounds according to Claim 17, characterized in that the structure-specific substances are antibodies, antibody fragments, agonists which bind specifically to receptors, cytokines, lymphokines, endothelins or antagonists thereof, other specific peptides and proteins, receptors, enzymes, enzyme substrates, nucleotides, ribonucleic acids, deoxyribonucleic acids, carbohydrates or lipoproteins.

20. Use of compounds according to Claim 17, characterized in that the ferromagnetic or ferrimagnetic substances are stable or quasi-stable colloidal particles of iron oxides, barium ferrite, strontium ferrite, pure iron, chromium dioxide, nickel, cobalt and iron oxides with manganese, copper, nickel or cobalt additives.

21. Use of agents in methods according to one of Claims 11 and 12, characterized in that they contain a plurality of ferromagnetic or ferrimagnetic substances with different coercive field strengths.

22. Use of the methods according to Claims 1-16 in fertility, histocompatibility, allergology, infectiology, hygiene, genetics, virology, bacteriology, toxicology, pathology, environmental analysis or medical diagnosis.

## Revendications

1. Procédé de détection qualitative et/ou quantitative d'analytes, caractérisé en ce que des substances colloïdales stables ou quasi stables ferromagnétiques ou ferrimagnétiques sont utilisées comme marquage magnétique à déceler dans des essais immunologiques hétérogènes et la magnétisation rémanente de l'échantillon est déterminée comme grandeur mesurée.

2. Procédé de détection qualitative et/ou quantitative d'analytes dans des essais immunologiques hétérogènes, caractérisé en ce que des marqueurs magnétiques liés opèrent au moment de la mesure dans son intégralité une magnétisation rémanente de l'échantillon pendant que la magnétisation, également dans l'échantillon de marqueurs magnétiques non liés présents, est diminuée par superparamagnétisme extrinsèque au moment de la mesure dans son intégralité.

3. Procédé de détection qualitative et/ou quantitative d'analytes dans des essais immunologiques hétérogènes, caractérisé en ce que
(i) d'abord des substances de structure spécifique qui se lient spécifiquement sur les analytes à détecter, sont marquées avec des substances colloïdales ferrimagnétiques ou ferromagnétiques, et puis
(ii) ces substances de structure spécifique magnétiquement marquées sont ajoutées à l'échantillon à mesurer;
(iii) l'échantillon à mesurer est remagnétisé au moyen d'un champ magnétique de force appropriée, installé de l'extérieur, et
(iv) après la mise hors circuit du champ extérieur, on mesure la rémanence de la magnétisation des particules colloïdales à l'aide de palpeurs de champ magnétique, la rémanence apparaissant par liaison spécifique et sa grandeur étant extraite pour l'analyse.

4. Procédé suivant la revendication 3, caractérisé en ce qu'on marque, au lieu des substances de structure spécifique, des analytes à déceler avec des substances ferrimagnétiques ou ferromagnétiques et on ajoute aux échantillons à mesurer les substances de structure spécifique, qui se lient spécifiquement sur les analytes à détecter.

5. Procédé suivant les revendications 3 ou 4, caractérisé en ce que les substances de structure spécifique sont des anticorps, des fragments d'anticorps, de la biotine ou des substances liant spécifiquement la biotine, de l'avidine ou de la streptavidine, des agonistes se liant spécifiquement sur des récepteurs ou leurs antagonistes, des peptides spécifiques et des protéines, des récepteurs, des enzymes, des substrats enzymatiques, des nucléotides, des acides ribonucléiques, des acides désoxyribonucléiques, des hydrates de carbone ou des lipoprotéines.

6. Procédé suivant la revendication 5, caractérisé en ce que les agonistes se liant spécifiquement sur des récepteurs ou les antagonistes sont des cytokines, des lymphokines, des endothélines ou leurs antagonistes.

7. Procédé suivant les revendications 3, 4, 5 ou 6, caractérisé en ce que les substances de structure spécifique ont une constante de liaison dans l'intervalle de 10⁵- 10¹⁵ (mole/l)⁻¹.

8. Procédé suivant l'une des revendications 1 à 7, caractérisé en ce que l'échantillon se déplace pendant la mesure et ainsi le signal d'échantillon est modulé.

9. Procédé suivant l'une des revendications 1 à 8, caractérisé en ce qu'on utilise comme palpeurs de champ magnétique, des bobines d'induction changées en gradiomètres, des magnétomètres discriminateurs de flux, des palpeurs de magnétorésistance géants ou des convertisseurs magnétorésistifs.

10. Procédé suivant l'une des revendications 1 à 8, caractérisé en ce qu'on utilise comme palpeurs de champ magnétique des interféromètres quantiques à supraconducteur (SQUID).

11. Procédé suivant les revendications 1 à 10, caractérisé en ce qu'on effectue une détermination simultanée de plusieurs analytes différents par magnétisation graduelle de l'échantillon à mesurer.

12. Procédé suivant la revendication 11, caractérisé en ce qu'on utilise différentes substances ferromagnétiques ou ferrimagnétiques avec des intensités discrètes de champ coercitif pour une détermination quantitative simultanée d'analytes.

13. Procédé suivant les revendications 1 à 12, caractérisé en ce que le temps de relaxation de Néel intrinsèque des substances ferromagnétiques et ferrimagnétiques utilisées est supérieur au temps de mesure.

14. Procédé suivant la revendication 13, caractérisé en ce que le temps de relaxation néelien des substances ferromagnétiques et ferrimagnétiques utilisées est supérieur à 10⁻⁴ seconde à 20°C.

15. Procédé suivant les revendications 1 à 14, caractérisé en ce que les substances ferromagnétiques et ferrimagnétiques présentent une taille particulaire dans l'intervalle de 1 à 1000 nm.

16. Procédé suivant les revendications 1 à 15, caractérisé en ce que les substances ferromagnétiques et ferrimagnétiques sont stabilisées par une enveloppe d'oligomères ou d'hydrates de carbone polymères, de protéines, de peptides, de nucléotides, de tensioactifs, de polymères synthétiques et/ou de lipides.

17. Utilisation de composés dans des procédés suivant les revendications 1 - 16, caractérisée en ce que les composés sont constitués de combinaisons de substances ferrimagnétiques ou ferromagnétiques stables ou quasi stables avec des substances de structure spécifique.

18. Utilisation de composés suivant la revendication 17, caractérisée en ce que les particules ferrimagnétiques ou ferromagnétiques présentent un temps de relaxation de Néel qui est supérieur à 10⁻⁴ seconde.

19. Utilisation de composés suivant la revendication 17, caractérisée en ce que les substances de structure spécifique sont des anticorps, des fragments d'anticorps, des agonistes se liant spécifiquement sur des récepteurs, des cytokines, des lymphokines, des endothélines ou leurs antagonistes, d'autres peptides et protéines spécifiques, des récepteurs, des enzymes, des substrats enzymatiques, des nucléotides, des acides ribonucléiques, des acides désoxyribonucléiques, des hydrates de carbone ou des lipoprotéines.

20. Utilisation de composés suivant la revendication 17, caractérisée en ce que les substances ferromagnétiques ou ferrimagnétiques sont des particules colloïdales stables ou quasi stables d'oxydes de fer, de ferrite de baryum, de ferrite de strontium, de fer pur, de dioxyde de chrome, de nickel, de cobalt, ainsi que d'oxydes de fer avec des additions de manganèse, de cuivre, de nickel ou de cobalt.

21. Utilisation de moyens dans des procédés suivant les revendications 11 ou 12, caractérisée en ce que qu'ils contiennent plusieurs substances ferromagnétiques ou ferrimagnétiques avec des intensités différentes de champ coercitif.

22. Utilisation des procédés suivant les revendications 1-16, dans la fertilité, l'histocompatibilité, l'allergologie, l'infectiologie, l'hygiène, la génétique, la virologie, la bactériologie, la toxicologie, la pathologie, l'analytique environnementale ou les diagnostics médicaux.
